Europäisches Patentamt

European Patent Office

Office européen des brevets

(19)

(11) Numéro de publication : **0 059 817**
**B1**

# (12) FASCICULE DE BREVET EUROPÉEN

(45) Date de publication du fascicule du brevet :
09.10.85

(21) Numéro de dépôt : **81400374.5**

(22) Date de dépôt : **11.03.81**

(51) Int. Cl.⁴ : **A 61 K 9/52**

(54) **Serpentine; alstonine et sempervirine, médicaments inhibant spécifiquement les cellules cancéreuses et tumorales.**

(43) Date de publication de la demande :
15.09.82 Bulletin 82/37

(45) Mention de la délivrance du brevet :
09.10.85 Bulletin 85/41

(84) Etats contractants désignés :
**AT BE CH DE FR GB IT LI LU NL SE**

(56) Documents cités :
FR-A- 1 347 413
FR-A- 2 419 722
FR-A- 2 419 725
FR-A- 2 450 607
GB-A- 737 310
GB-A- 793 808

(73) Titulaire : **Beljanski, Mirko**
**46, boulevard de Port Royal**
**F-75005 Paris (FR)**

**Bugiel, Jean**
**72, boulevard Saint Marcel**
**F-75005 Paris (FR)**

(72) Inventeur : **Beljanski, Mirko**
**46, boulevard de Port Royal**
**F-75005 Paris (FR)**
Inventeur : **Bugiel, Jean**
**72, boulevard Saint Marcel**
**F-75005 Paris (FR)**

(74) Mandataire : **Pinguet, André**
**CAPRI 28 bis, avenue Mozart**
**F-75016 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention concerne des produits utilisables comme médicaments comportant de la serpentine, alstonine, sempervirine et flavopéreirine à action préférentielle sur la réplication des ADN et la synthèse des ARN des cellules tumorales, cancéreuses et vitales.

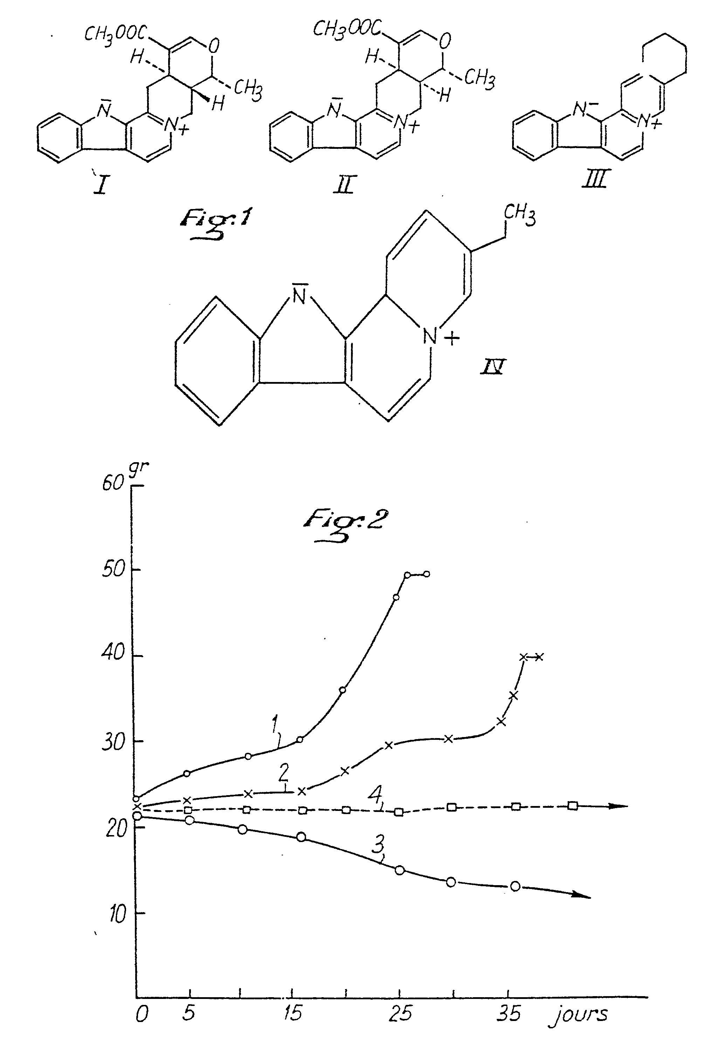

Une substance anti-cancéreuse, anti-tumorale vraie doit distinguer les cellules tumorales des cellules saines, et tuer les premières, sans porter préjudice aux secondes. On a trouvé que quatre alcaloïdes extraits de végétaux et appartenant au groupe des béta-carbolines quaternaires, possèdent ces propriétés anti-cancéreuses, anti-tumorales, ou antivirales, et peuvent être utilisés comme médicaments pour le traitement des cancers chez les humains en particulier s'ils sont administrés sous une forme galénique à libération prolongée. Ce sont : la serpentine, de formule I, alcaloïde du Rauwolfia serpentina, qui peut être extraite à l'état pur de cette plante ; l'alstonine, de formule II, alcaloïde de diverses variétés de Rauwolfia (obscura, vomitoria) de Vinca rosea et autres apocynacées qui peut être extraite de Rauwolfia obscura ; la sempervirine, de formule III, alcaloïde de gelsemium sempervirens (jasmin jaune de Caroline) qui peut être synthétique et la flavopéreirine, alcaloïde du Geissospernum laeve (Vellozo) Baillon, apocynacées de formule IV qui peut être synthétisé. Les formules I, II, III et IV de ces quatre alcaloïdes sont représentées ci-après dans la figure 1.

Il a été démontré que les quatre alcaloïdes selon l'invention ont une activité anti-cancéreuse vraie, c'est-à-dire qu'ils détruisent les cellules cancéreuses, mais ne détruisent pas les cellules saines.

Des cultures de cellules saines (cellules primaires de reins de singe, cellules de lignée « Vero » et « SIRC ») et des cultures de cellules cancéreuses (cellules K.B., cellules Hela, cellules Hep II et cellules L) ont reçu une dose de 200 µg, soit de serpentine, soit d'alstonine, soit de sempervirine, par ml de culture. Les résultats sont résumés dans le tableau I ci-après : toutes les cellules cancéreuses étaient détruites, aussi bien par la serpentine, que par la sempervirine en 24 à 48 heures ; par contre, il n'y avait aucune destruction des cellules saines au bout de huit jours d'observation.

L'effet cytotoxique spécifique, vis-à-vis des cellules tumorales utilisées, ne s'est jamais démenti au cours des très nombreuses expériences effectuées. Par contre, pour effectuer les cellules saines il a fallu des quantités d'alcaloïdes au moins dix fois plus grandes (plus de 2 mg/ml). (Les cultures de cellules in vitro sans mycoplasmes ont été effectuées dans les conditions classiques et en l'absence d'antibiotique qui pourraient fausser les résultats).

L'activité anti-cancéreuse des quatre alcaloïdes selon l'invention a été confirmée par des expériences pharmacologiques sur des souris atteintes de différents types de cancers.

Action sur le cancer mammaire greffable de la souris :

Vingt souris de 18 à 20 g, de souche RhO III ont été inoculées, par voie sous-cutanée, par 0,2 ml d'un extrait de tumeur MS 301, broyé. Dix souris ont été gardées comme témoins ; dix autres recevaient, le troisième jour après l'inoculation, 400 µg d'alstonine au niveau de l'implantation tumorale, deux fois par jour, pendant quinze jours. Au seizième jour, les souris étaient anesthésiées et sacrifiées, les tumeurs excisées et pesées.

La même expérience fut effectuée avec la serpentine au lieu d'alstonine.

Les résultats sont donnés dans le tableau II ci-dessous :

Tableau II

| POIDS MOYEN (en grammes) DES TUMEURS, AU 16è JOUR | | |
|---|---|---|
| Souris témoins | Souris traitées par la serpentine | Souris traitées par l'alstonine |
| 3,2 (compris entre 2,6 et 3,8)<br><br>Signification statistique p : 0,01 | 1,6 (compris entre 0,9 et 2,5) | 1,7 (compris entre 0,8 et 2,6) |

Activité vis-à-vis du lymphome YC 8 de la souris :

Trente souris BALB C de 17 à 20 g sont inoculées par voie intrapéritonéale par $10^3$ cellules ascitiques du lymphome YC 8 de la souris fraîchement prélevées.

Dix souris sont gardées comme témoins.

2

Dix souris sont traitées, le 3e jour après l'inoculation, par l'alstonine : deux fois par voie intrapéritonéale et pendant huit jours consécutifs (400 μg par jour).

Dix souris sont traitées le 3e jour après l'inoculation, par un mélange alstonine + cyclophosphamide.

Dans ces expériences l'alstonine peut être remplacée par la serpentine. Les résultats sont présentés dans le tableau III ci-après et illustrent la survie des animaux pendant trois mois (une survie de quelques jours n'est pas prise en considération).

Chez les souris témoins la tumeur ascitique se développe en 20-22 jours environ et la souris atteint alors un poids de 32-36 g. Les quatre souris ayant survécu au traitement par l'alstonine ont plus de trois mois. Elles sont tout à fait normales et leur poids initial a faiblement et normalement progressé (23-24 g). Lorsqu'on applique, par voie intra-péritonéale, un traitement par un mélange d'alstonine et de cyclophosphamide (doses par jour et par souris de 400 μg d'alstonine et 2 mg de cyclophosphamide) neuf sur dix souris survivent parfaitement sans amaigrissement ni récidive (Tableau III ci-après).

Tableau III

| TEMPS DE SURVIE DES SOURIS TEMOINS ET TRAITEES | | | |
|---|---|---|---|
| Jours de survie | Témoins | Traitées par l'alstonine | Traitées par alstonine + cyclophosphamide |
| 0 | 10/10 | 10/10 | 10/10 |
| 22 | 0/10 | 7/10 | 9/10 |
| 60 | – | 4/10 | 9/10 |
| 90 | – | 4/10 | 9/10 |

Par contre, lorsque le cyclophosphamide seul est administré, on constate que les souris sont guéries, mais ceci n'est qu'une apparence car après 1-3 mois, l'amaigrissement est tel qu'elles meurent et que chez d'autres l'apparition de nouvelles tumeurs s'observe.

Il faut noter que des souris porteuses de lymphomes YC 8 et traitées par l'alstonine seule, aux mêmes doses par voie sous-cutanée, ne survivent pas plus longtemps que les souris témoins.

Par contre, l'administration simultanée par voie sous-cutanée d'alstonine et de cyclophosphamide, pendant 4 à 5 jours consécutifs, se traduit par une survie parfaite de toutes les souris traitées (6/6).

Activité vis-à-vis des tumeurs « Ascite d'Ehrlich » chez la souris

$5 \times 10^3$ cellules Ascite d'Ehrlich sont inoculées à des souris blanches Swiss de 20-24 g, par voie intrapéritonéale.

Divers traitements furent appliqués dès le quatrième ou le cinquième jour et pendant dix à quinze jours. Nous pouvons conclure que si le cyclophosphamide injecté seul, par voie intrapéritonéale ou sous-cutanée, ni l'alstonine seule, ni la serpentine seule, ne peuvent définitivement arrêter la tumeur ascitique d'Ehrlich. Un net ralentissement du développement des tumeurs est observé, mais les tumeurs repartent après arrêt du traitement dans le cas du cyclophosphamide seul, le redémarrage peut être plus tardif qu'avec la serpentine ou l'alstonine. Par contre, l'association de serpentine (400 μg par jour) et de cyclophosphamide (2 mg par jour) par voie I.P. permet d'obtenir une survie définitive (survie définitive : survie normale pendant trois mois sans perte de poids) et le pourcentage des survivants varie de 80 à 100 % (tableau IV ci-après). Les souris ainsi guéries augmentent normalement de poids et après quatre à six mois celui-ci est de 24-26 g. Leur aspect est absolument normal et rien ne les distingue de souris saines. (Dans les expériences, seule la survie sans récidive est prise en considération).

Tableau IV

| SURVIE DES SOURIS PORTEUSES DE TUMEURS ASCITIQUES D'EHRLICH | | | | |
|---|---|---|---|---|
| Jours de survie | Témoins | Traitées par la serpentine (I.P.) | Traitées par le cyclophosphamide (I.P.) | Traitées par Serpentine + cyclophosphamide (I.P.) |
| 0 | 10/10 | 10/10 | 6/6 | 10/10 |
| 25 | 0/10 | 10/10 | 6/6 | 10/10 |
| 35 | - | 0/10 | 6/6 | 9/10 |
| 60 | - | - | 2/6 | 9/10 |
| 120 | - | - | 0/6 | 9/10 |

La figure 2, où sont portés en abscisses le nombre de jours de traitement et en ordonnées le poids (en gramme) des souris, montre l'évolution de la tumeur d'Ehrlich chez les souris traitées, soit par la serpentine seule (courbe 2), soit par le cyclosphosphamide seul (courbe 3), soit par la serpentine + cyclophosphamide (courbe 4) par voie intrapéritonéale et aux doses indiquées ci-dessus. Les deux traitements, soit par la serpentine, soit par le cyclophosphamide seul, permettent d'obtenir un net ralentissement du poids des tumeurs avec toutefois un amaigrissement très fort de la souris traitée par le cyclophosphamide seul. Aucun de ces deux traitements ne permet d'obtenir une survie définitive. Par contre, le mélange de serpentine et de cyclophosphamide permet d'obtenir une survie définitive.

Inoculées par voie sous-cutanée, aux doses indiquées ci-dessus, et séparément, l'alstonine et la serpentine sont sans effet sur la survie définitive des souris, alors que l'association alstonine (ou serpentine) + cyclophosphamide par voie sous-cutanée, provoque l'arrêt de l'évolution de la tumeur et la survie des souris ainsi traitées est normale.

Activité vis-à-vis de l'ascite solide d'Ehrlich de la cuisse, chez la souris :

Le muscle interne de la souris de souche Swiss décroît $10^6$ cellules de l'ascite d'Ehrlich par voie intramusculaire. Le traitement n'est commencé que lorsque la tumeur est apparente. La serpentine, ou l'alstonine, est administrée seule par voie intramusculaire dans la région de la tumeur aux doses de 0,8 mg par jour et pendant dix jours consécutifs. Après 26 jours, les souris sont sacrifiées, les tumeurs excisées et pesées. On constate que l'alstonine (ou la serpentine) réduit le poids moyen des tumeurs, de l'ordre de 40 à 50 % (comme on peut le voir d'après le tableau V ci-après), ce qui, lorsque les souris ne sont pas sacrifiées se traduit par une survie de quelques jours seulement.

Par contre, l'administration d'alstonine (ou de serpentine) et de cyclophosphamide dans la tumeur elle-même (400 µg d'alstonine + 2 mg de cyclophosphamide) par jour et pendant 10 jours consécutifs, conduit à la nécrose de la tumeur. La souris retrouve une mobilité normale. Le taux de survie des souris ainsi traitées est de l'ordre de 80 %. Lorsque le traitement est arrêté trop tôt (avant 5 jours) quelques souris présentent une récidive. De nouvelles injections de serpentine (ou alstonine) + cyclophosphamide, permettent d'arrêter définitivement le développement de la tumeur.

0 059 817

Tableau V

Régression de la tumeur solide d'Ascite d'Ehrlich

| POIDS MOYEN DES TUMEURS (en gramme) | | |
|---|---|---|
| Souris témoins | Souris traitées par l'alstonine | Souris traitées par alstonine + cyclo-phosphamide |
| Poids moyen 11,4 ; compris entre 10,4 et 12,4<br><br>p : 0,01 | Poids moyen 7,8 ; compris entre 5,5 et 10,4 | Poids moyen 0,07 ; compris entre 0,04 et 0,11 |

Les expériences présentées ont été répétées plusieurs fois.

Par ailleurs, l'effet cytotoxique spécifique de la sempervirine sur des cellules cancéreuses cultivées in vitro a été confirmé sur des souris porteuses de cellules cancéreuses et traitées par cet alcaloïde.

On décrit ci-après à titre d'exemple l'activité de la semperverine vis-à-vis du lymphome YC 8 de la souris.

34 souris BALB c de 17-20 g sont inoculées par voie intrapéritonéale par $10^3$ cellules ascitiques du lymphome YC 8 de la souris fraîchement prélevées et dix souris sont gardées comme témoins.

24 souris sont traitées le deuxième jour après inoculation par la sempervirine dissoute en milieu tamponné (pH 7,65) à raison de deux fois par jour par voie intrapéritonéale (300 µg par jour) et pendant 10 jours consécutifs.

Les résultats sont présentés dans le tableau IX ci-après et illustrent la survie pendant trois mois des animaux traités. (Une survie de plusieurs jours n'est pas prise en considération).

Temps de survie des souris témoins et traitées

(Tableau IX)

| Jours de survie | Témoins | Traitées par la sempervirine |
|---|---|---|
| 0 | 10/10 | 24/24 |
| 20 | 0/10 | 24/24 |
| 60 | 0/10 | 20/24 |
| 90 | 0/10 | 20/24 |

Chez les souris témoins, la tumeur ascitique se développe en 20-22 jours environ, et la souris atteint un poids de 34-38 g.

Les souris ayant survécu au traitement par la Sempervirine ont été gardées plus de trois mois. Les résultats sont indépendants du sexe des souris utilisées.

Les souris survivantes sont tout à fait normales et leur poids initial a normalement progressé. Il faut noter que des souris porteuses de lymphome YC 8 et traitées par la sempervirine par voie sous-cutanée ne survivent pas très longtemps après les souris témoins.

De même, on a étudié l'activité de la sempervirine vis-à-vis des tumeurs Ascite d'Ehrlich de la souris :

30 souris BALB c de 17-20 g ont été inoculées par voie intrapéritonéale par $5 \times 10^3$ cellules d'Ascite d'Ehrlich, fraîchement prélevées.

10 souris ont été gardées comme témoins et 20 souris ont été traitées le deuxième jour après l'inoculation par la sempervirine à raison de deux fois par jour par voie intrapéritonéale (300 µg par jour et pendant 10 jours consécutifs).

5

# 0 059 817

Les résultats sont présentés dans le tableau X ci-après et illustrent la survie des animaux pendant trois mois, une survie de plusieurs jours n'étant pas prise en considération.

Temps de survie des souris témoins et traitées

(Tableau X)

| Jours de survie | Témoins | Traitées par la sempervirine |
|---|---|---|
| 0 | 10/10 | 20/20 |
| 21 | 1/10 | 15/20 |
| 60 | 0/10 | 10/20 |
| 90 | 0/10 | 10/20 |

Les résultats montrent que le traitement par la sempervirine seule permet une guérison définitive des souris porteuses de cellules d'Ascite d'Ehrlich dans une proposition qui se situe autour de 50 %. Les mêmes résultats ont été obtenus lorsque les souris blanches SWISS ont été utilisées dans ce même type d'expérience.

On a par ailleurs étudié l'action de la flavopéreirine sur les cellules saines et sur les cellules cancéreuses cultivées in vitro.

Les cellules en culture ont reçu une dose de 150 µg de flavopéreirine par ml de culture ; les cellules cancéreuses utilisées sont détruites en 24-48 heures, par contre les cellules saines ne sont pas détruites pendant les 7 jours d'observation.

| Cellules normales : (tableau VI) | | |
|---|---|---|
| - Cellules primaires de reins de singe | - + 150 µg de fla- vopéreirine dès l'ensemencement | - Pas de destruc- tion pendant 7 jours |
| - Cellules "Vero" (cellules de lignée) | - + 150 µg de fla- vopéreirine dès l'ensemencement | - Pas de destruc- tion pendant 7 jours |
| Cellules cancéreuses : | | |
| - Cellules KB | - + 150 µg de fla- vopéreirine dès l'ensemencement | - Destruction to- tale en 24-48 heures |
| - Cellules Hela (culture de 3 jours) | - + 150 µg de fla- vopéreirine dès l'ensemencement | - Destruction to- tale en 24-48 heures |
| - Cellules L | - + 150 µg de fla- vopéreirine dès l'ensemencement | - Destruction to- tale en 24-48 heures. |

6

Les cellules cancéreuses et saines utilisées ne contenaient pas de mycoplasmes et le milieu de culture ne contenait pas d'antibiotique, ces derniers pouvant sensibiliser les cellules et par conséquent fausser les résultats.

L'activité anticancéreuse de la flavopéreirine a été confirmée par des expériences sur des souris atteintes soit par les cellules ascitiques de lymphome YC 8, soit sur des souris atteintes de tumeur ascitique d'Ehrlich.

En ce qui concerne l'activité de la flavopéreirine vis-à-vis du lymphome YC 8 de la souris, 30 souris BALB c de 18-20 g ont été inoculées par voie intrapéritonéale par $10^4$ cellules ascitiques du lymphome YC 8 fraîchement prélevées et en pleine division chez la souris.

Dix souris ont été gardées comme témoins et 20 souris ont été traitées 24 heures après inoculation.

La flavopéreirine est dissoute en milieu physiologique tamponné (pH 7,65) et deux injections par voie intrapéritonéale et par jour sont administrées (2 × 150 μg de flavopéreirine) et ceci pendant 10 jours consécutifs.

Les résultats présentés dans le tableau VII ci-dessous illustrent la survie des animaux traités :

Temps de survie des souris témoins et traitées

(Tableau VII)

| Jours de survie | Témoins | Traitées par la flavopéreirine |
|---|---|---|
| 0 | 10/10 | 20/20 |
| 2I | 0/10 | 20/20 |
| 60 | 0/10 | 17/20 |
| 90 | 0/10 | 17/20 |

Toutes les souris qui ont survécu au traitement par la flavopéreirine (voie I.P.) ne pouvaient se distinguer de souris de laboratoire non inoculées par des cellules tumorales, leur développement étant tout à fait normal pendant plus de trois mois d'observation.

L'administration de flavopéreirine par voie sous-cutanée ralentit le développement des cellules tumorales (lymphome YC 8) et la survie de trois mois concerne environ 70-80 % des souris traitées à raison de deux fois par jour (2 × 150 μg) et pendant 10 jours consécutifs.

On a constaté que le poids des souris témoins augmente essentiellement à partir du 10-12e jour et vers le 20-21e jour, les souris atteignent un poids de 34-37 g.

En ce qui concerne l'activité de la flavopéreirine, vis-à-vis des tumeurs ascites d'Ehrlich de la souris, 30 souris SWISS de 20-22 g ont été inoculées par voie intrapéritonéale par 5 × $10^3$ cellules ascite d'Ehrlich fraîchement prélevées.

Dix souris ont été gardées témoins et 20 souris ont été traitées 24 heures après l'inoculation par la flavopéreirine à raison de deux fois par jour (2 × 150 μg) par voie intrapéritonéale et pendant 10 jours consécutifs.

Les résultats du tableau VIII ci-après illustrent la survie des animaux pendant trois mois (une survie de plusieurs jours n'est pas prise en considération).

Temps de survie des souris témoins et traitées

(Tableau VIII)

| Jours de survie | Témoins | Traitées par la flavopéreirine |
|---|---|---|
| 0 | 10/10 | 20/20 |
| 24 | 0/10 | 17/20 |
| 60 | 0/10 | 11/20 |
| 90 | 0/10 | 11/20 |

Les résultats montrent que la flavopéreirine utilisée seule permet la guérison d'un important pourcentage de souris dont la vie est tout à fait normale et ceci pendant les trois mois d'observation. Les mêmes résultats ont été obtenus lorsque les souris BALBc ont été utilisées dans ce même type d'expérience.

Par ailleurs, on a étudié l'action anti-virale des alcaloïdes selon l'invention.

On a étudié l'activité de la sempervirine vis-à-vis du virus du fibrome de Shope du lapin de la façon suivante :

Le stock de virus de fibrome de Shope a été préparé à partir des testicules de lapins injectés in vivo et conservés sous forme lyophilisée.

Le titre a été évalué en inoculant les dilutions de virus par voie intradermique et en observant la grosseur des tumeurs.

Des lapins blancs (Australie, Bouska) de 2,5 à 3,5 kg ont eu les deux flancs rasés. Après désinfection de la peau, le virus (10 DI 50 sans 0,2 ml) a été injecté par voie intradermique en 4 points par côté.

Le traitement par la sempervirine a toujours été effectué dans les sites du flanc droit, 24 heures après l'inoculation du virus à raison d'une fois par jour et pendant trois jours de suite.

Ches les lapins témoins, apparaît au troisième ou quatrième jour, aux sites d'inoculation, une papule qui s'agrandit pour atteindre sa grosseur maximale (diamètre 1-1,5 cm) vers le sixième ou huitième jour.

Au dixième jour, on observe un début de nécrose qui sera totale au quinzième jour et la tumeur disparaît progressivement. Chez les lapins dont uniquement les sites d'inoculation du virus du flanc droit ont été traités par la sempervirine, les papules n'apparaissent pas lorsque les doses de 200 μg à 800 μg par point d'injection ont été administrées. Chez ces mêmes lapins, les papules se développent normalement sur le flanc du lapin, côté non traité.

Des expériences préliminaires ont été réalisées avec le virus de la vaccine (neurovaccine lyophilisée), titré par inoculation intradermique.

Des petites papules apparaissent 48 heures après inoculation intradermique du virus. La taille des papules n'augmente pas après 4 jours.

L'injection de sempervirine (100-400 μg), 24 heures après l'inoculation dans cette même région se traduit par la non-apparition de papules ou apparition de papules dont la taille est considérablement diminuée.

La sempervirine est active contre la multiplication de virus du type de celui du fibrome de Shope et de la vaccine.

On a étudié l'activité de la flavopéreirine vis-à-vis du virus du fibrome de Shope et de la vaccine du lapin de la façon suivante.

Le virus du fibrome de Shope, gardé sous forme lyophilisée, est dilué et son titre évalué en pratiquant des injections intradermiques sur les deux flancs rasés des lapins (lapins blancs d'Australie, Bouska) de 3,0 à 3,5 kg).

Le virus a été injecté en quatre points par côté.

Le traitement par voie intradermique, par la flavopéreirine a toujours été effectué dans les sites du flanc droit, 24 heures après l'inoculation du virus : une fois par jour et pendant quatre jours de suite.

Chez les lapins témoins apparaît au troisième ou quatrième jour, aux sites d'inoculation, une papule qui s'agrandit pour atteindre sa grosseur maximale de 1,5 cm de diamètre vers le huitième jour.

Au dixième jour, la nécrose de la tumeur commence et devient totale ; vers le quinzième jour : la tumeur n'existe plus.

Chez les lapins traités dont uniquement les sites d'inoculation du virus du flanc droit ont été traités par la flavopéreirine, les papules n'apparaissent pas lorsque les doses de 150-600 μg par point d'injection ont été quotidiennement adminitrées.

En conclusion, la flavopéreirine (200-600 μg) administrée par voie intradermique dans la région où le virus de la vaccine a été inoculé, empêche l'apparition et le développement des papules virales et la flavopéreirine est active contre les virus du type de ceux du fibrome de Shope et de la vaccine.

D'autre part, on a étudié ci-après l'activité de la sempervirine, flavopéreirine, alstonine et serpentine, contre le virus de la grippe :

Une suspension de stock du virus de la grippe, après dilution convenable est inoculée par voie nasale à 45 souris SWISS de 17-19 g.

Dans le groupe A, 15 souris ont été gardées comme témoins et dans le groupe B, 15 souris ont été traitées dès l'inoculation virale par la sempervirine : 200 μg par voie intrapéritonéale, deux fois par jour (soit 400 μg par jour) et pendant 8 jours consécutifs.

Dans le groupe C, 15 souris ont été traitées 24 heures après l'inoculation virale comme les souris du groupe B.

Dix jours après l'inoculation, 5 souris de chaque groupe ont été anesthésiées et sacrifiées, et l'on procède à un examen macroscopique de chaque lobe des poumons. Le degré de congestion a été noté de 0 à 5 croix.

Les résultats du tableau XI montrent que chez les témoins, cliniquement malades, la moitié des lobes sont atteints par le virus (congestion) et chez les traitées, apparemment bien portantes, 0 ou 1 lobe par poumon atteint, montrant un très net ralentissement ou même une inhibition notable de la multiplication du virus de la grippe par la sempervirine.

8

Des résultats tout à fait comparables peuvent être obtenus si au lieu de sempervirine, on utilise à des doses comparables soit la flavopéreirine, soit la serpentine, soit l'alstonine.

Tableau XI

| Jours de survie | Témoins Groupe A | Traitées par la sempervirine | |
|---|---|---|---|
| | | Groupe B | Groupe C |
| 0 | 10/10 | 10/10 | 10/10 |
| 10 | 8/10 | 10/10 | 10/10 |
| 16 | 2/10 | 8/10 | 6/10 |

Les souris témoins sont toutes cliniquement malades.

On a étudié la toxicité des β-carbolines quaternaires objets de l'invention. Par les 4 substances étudiées on a trouvé une DL50 comprise entre 335 et 450 mg par kg chez le rat par voie intrapéritonéale. Chez le chien, la toxicité par voie veineuse (en perfusion) est de l'ordre de 70 à 100 mg par kg et par injection flash de l'ordre de 50 mg par kg pour une dose maximum tolérée de l'ordre de 20 mg par kg, toujours par voie veineuse.

Dans les expériences pharmacologiques, chez les souris porteuses de tumeurs (soit l'ascite d'Ehrlich, soit le lymphome YC 8) une dose journalière de serpentine ou alstonine de 400 à 800 μg par voie intrapéritonéale, est facilement tolérée pendant 15 jours successifs. La même tolérance est observée lorsque les mêmes doses sont administrées par injections intramusculaires, ou par voie buccale.

Il faut souligner d'autre part que la serpentine et l'alstonine sont éliminées moins de trois heures après l'injection de 800 μg par voie intraveineuse.

Par contre, dans l'association β-carboline quaternaire + cyclophosphamide l'alcaloïde n'est éliminé qu'au bout de 18 heures.

Chez les souris la destruction des cellules cancéreuses et tumorales est obtenue par administration d'un mélange, préparé, de serpentine, d'alstonine ou flavopéreirine et de cyclophosphamide.

L'alstonine, la serpentine, la sempervirine et la flavopéreirine peuvent être utilisées dans le traitement des cancers chez les humains et en particulier dans le traitement des cancers du sein. Ces produits peuvent être administrés par voie intrapéritonéale, par voie intramusculaire, par voie intraveineuse ou par voie buccale, de préférence, en association avec le cyclophosphamide ou d'autres médicaments anticancéreux.

Par voie I.M. et I.V. on utilise de préférence une association de 100 mg de β-carboline quaternaire et de 10 mg de cyclophosphamide, cette dose administrée une fois par jour, et le traitement pouvant être poursuivis plusieurs mois. Par voie orale, on peut administrer 50 mg de β-carboline quaternaire + 5 mg de cyclophosphamide, une fois par jour, pendant plusieurs mois.

Toutefois, on a pu constater au cours des essais cliniques sur l'humain que les β-carbolines quaternaires avaient une demi-vie trop courte pour être administrée sous forme galénique mais que, de façon surprenante, on obtenait d'excellents résultats dans le traitement des cancers chez les humains en utilisant une nouvelle forme galénique constituée de microgranules à libération prolongée des β-carbolines quaternaires. Ces microgranules comprennent une âme neutre constituée d'un grain excipient inerte par exemple un ou plusieurs constituants tels que le sacchorose, l'amidon, le talc, la silice desséchante, le lactose et l'acide stéarique, le grain neutre étant muni d'une première couche comprenant la β-carboline quaternaire puis d'une seconde couche extérieure constituée par une enveloppe microporeuse comprenant au moins un polymère naturel et/ou synthétique appartenant à la classe constituée par la gomme laque, la gomme arabique, la gélatine, l'éthylcellulose, l'acétophtalate de cellulose, le triacétate de cellulose, le polyoxyéthylèneglycol, les méthacrylates, le copolymère styrènea-crylonitrile et la polyvinylpyrrolidone.

Des compositions telles que décrites ci-dessus, utiles comme agents anti-tumoraux, sont caractérisées en ce qu'elles sont associées avec une substance anti-tumorale classique de cyclophosphamide selon une proposition comprise entre 1 et 2 parties en poids pour 10 parties en poids de β-carboline quaternaire.

Exemple de préparation de la nouvelle forme galénique

On indique ci-après l'exemple de fabrication correspondant à 100 000 gélules dosées à 50 mg de β-carboline quaternaire.

A) Formule de fabrication

β-carboline quaternaire ... 5 kg
Saccharose — amidon — gomme laque — talc — silice desséchante — acide stéarique 24,300 kg
Alcool éthylique absolu q.s.

B) Procédé de préparation

On granule l'amidon de maïs et du saccharose puis on tamise et on turbine longuement les grains de façon à les rendre parfaitement sphériques. On tamise à nouveau et on sèche parfaitement.

Dans un mélangeur en acier inoxydable, on projette sur les âmes neutres ainsi obtenues une solution alcoolique de β-carboline quaternaire.

On réalise ensuite la première couche en incorporant à ces microgranules les autres excipients à l'exception de la gomme laque puis on recommence la pulvérisation de β-carboline quaternaire, cet enrobage étant recommencé plusieurs fois avec tamisage et séchage si nécessaire entre chaque couche.

Lorsque la première couche contenant le principe actif est terminée, on réalise la couche extérieure microporeuse, en projetant sur les granules la gomme laque en solution dans l'alcool éthylique absolu.

On sèche soigneusement en éliminant l'alcool éthylique restant, on tamise à nouveau et on contrôle comme ci-après le titre des microgranules obtenus avant de mettre en gélules, après avoir ajusté éventuellement le titrage par addition et homogénéisé avec des microgranules neutres pour arriver au titrage désiré de 50 mg de β-carboline quaternaire.

Mesure de la libération de la β-carboline quaternaire

La caractéristique des pores de l'enveloppe extérieure est choisie de façon à assurer une libération prolongée de β-carboline quaternaire théorique.

1ère heure : libération inférieure à 40 %
4e heure : libération inférieure à 75 %
8e heure : libération supérieure à 80 %

Pour contrôler cette caractéristique, on utilise un appareil à délitement dans lequel on met en contact une quantité de microgranules correspondant à environ 50 mg de principe actif avec des liquides artificiels, l'appareil permettant de maintenir une agitation constante et une température constante de 37 °C ± 5 °C. Les liquides artificiels sont des solutions tamponnées à pH successifs utilisées selon le schéma ci-dessous :

| Solutions | Temps de libération | pH | Résultats Théoriques | Réels |
|-----------|---------------------|-----|------------|-------|
| 25 ml liquide gastrique | 1 heure (1ère heure) | 1,5 | < 40% | 25% |
| 25 ml liquide intestinal | 1 heure (2ème heure) | 4,5 | > 40% | 41% |
| 25 ml liquide intestinal | 2 heures (3ème et 4ème heures) | 6,9 | < 75% | 64% |
| 25 ml liquide intestinal | 2 heures (5ème et 6ème heures) | 6,9 | > 75% | 78% |
| 25 ml liquide intestinal | 2 heures (7ème et 8ème heures) | 7,2 | > 80% | 91% |

Mis sous forme de microgranules, à libération prolongée, on n'a pas pu déterminer de DL50 chez le rat de sorte que, puisque aucune toxicité n'était à craindre des β-carbolines objets de l'invention, sauf à des doses très élevées, on a pu procéder à des essais cliniques selon des doses journalières pour une administration par voie orale comprise entre 2 et 500 mg et de préférence entre 50 et 200 mg et plus précisément 100 mg par jour.

A titre d'exemple, on a reproduit ci-après trois cas significatifs relatifs à des cancers du sein et pour lesquels on a utilisé la serpentine préparée selon la nouvelle forme galénique objet de l'invention décrite ci-dessus.

1. Madame M. a un cancer du sein gauche d'apparition récente et un squirrhe datant de plus de deux ans du sein droit.

En milieu hospitalier, on commence des irradiations préopératoires avec un pronostic pessimiste de trois mois de survie.

Le traitement par la serpentine commence après irradiation à raison de 100 mg en microgranules par jour per os en deux prises, l'une le matin, l'autre le soir. Un mois après, on constate une régression des deux tumeurs et devant le résultat, l'hôpital sursoit l'opération.

Le traitement à la serpentine est poursuivi pendant 6 mois au cours desquels progressivement la tumeur du sein diminue et disparaît alors que le squirrhe lui-même régresse considérablement.

Après ces six mois, le traitement est poursuivi de façon réduite six autres mois (un mois sur deux). Depuis lors, la malade se porte bien. Il reste une cicatrice souple du squirrhe mais plus aucune masse suspecte ni à droite ni à gauche.

2. On découvre chez Madame P. une tumeur du sein gauche cliniquement et radiologiquement maligne, confirmée par une ponction biopsique.

On instaure une thérapeutique radiothérapique dans un but opératoire, le chirurgien ayant décidé l'amputation large du sein. Mais la malade refuse l'intervention et n'accepte qu'une exérèse localisée de la tumeur palpable.

C'est alors que Madame P. commence le traitement à la serpentine en microgranules durant trois mois à la dose de 100 mg par jour en deux prises matin et soir per os.

Au cours de ces trois mois, la douleur qui persistait après l'intervention disparaît progressivement et le sein prend un aspect normal et indolore.

Tous les trois mois, elle a eu 15 jours de traitement par la serpentine pendant un an. Depuis lors, sans traitement, la malade se porte parfaitement bien. Ses examens de sang sont normaux. Elle a subi une intervention pour la surdité sans aucune complication. Par ailleurs, elle continue sa vie professionnelle normalement.

3. Madame L., opérée dix ans auparavant d'un cancer du sein, fait une sciatique, qui se révèle être secondaire et la métastase sacrée.

Malgré la radiothérapie et traitement chimiothérapique, des métastases osseuses multiples apparaissent à la colonne vertébrale, au fémur, au calcaneum droit, aux côtes, à l'humérus gauche.

Une fracture spontanée du col du fémur apparaît. C'est alors qu'est instauré un traitement à la serpentine en microgranules à libération prolongée.

La fracture du col du fémur se consolide ainsi que disparaît la métastase calcanéenne, ce qui permet après 6 mois de traitement à la malade de remarcher.

Les autres métastases osseuses sont enrayées tandis qu'avec la serpentine est continué un traitement à l'endoxan et au méthotrexate, grâce à un produit nouveau leucostimulant.

Aucune métastase organique n'est constatée, mais une métastase crânienne apparaît provoquant des crises d'épilepsie Bravaus-jacksonienne au cours desquelles la malade meurt.

Les trois cas ci-dessus cités à titre d'exemple ont été soumis à un traitement à la serpentine.

Les deux premiers ont abouti à une guérison clinique et la troisième a permis de stopper une évolution suraiguë des métastases, d'aboutir à une consolidation de fractures et à une survie.

Des essais comparables ont été menés avec les autres β-carbolines montrant des actions comparables sur les cellules tumorales, cancéreuses et virales (virus à ADN ou à ARN) aussi bien par voie orale, intraveineuse, intramusculaire ou intrapéritonéale.

On peut donc conclure à une identité de nature d'action pour les produits objets de la présente invention.

(Voir tableau I page 12)

## Tableau I

Action de la serpentine, de l'alstonine de la sempervirine et de la flavopéreirine sur les cellules en culture,
provenant de tissus sains et de tissus cancéreux.

| | | | |
|---|---|---|---|
| **Sur cellules normales** | | | |
| Cellules primaires de reins de singe | en culture de 7 jours dès l'ensemencement | + 200 μg de serpentine/ml, ou alstonine ou sempervirine ou flavopéreirine | Pas de destruction pendant 8 jours |
| Cellules "Vero" (cellules de lignée) | en culture de 3 jours dès l'ensemencement | | "          " |
| Cellules "SIRC" (cellules de lignée) | en culture de 3 à 5 jours dès l'ensemencement | | "          " |
| **Sur cellules cancéreuses** | | | |
| Cellules K.B. | en culture de 3 jours dès l'ensemencement | + 200 μg de serpentine/ml, ou alstonine ou sempervirine ou flavopéreirine | Destruction totale en 24-48 heures |
| Cellules Hela | en culture de 3 jours dès l'ensemencement | | "          " |
| Cellules Hep II | en culture de 3 jours dès l'ensemencement | | "          " |
| Cellules L | en culture de 3 jours dès l'ensemencement | | "          " |
| Neuroblastome Neuroblastome Tératome | tumeurs humaines en culture primaire | | "          " |

0 059 817

**Revendications**

1. Compositions pharmaceutiques à action préférentielle sur la réplication des ADN et la synthèse des ARN des cellules tumorales, cancéreuses et virales, caractérisées en ce qu'elles sont constituées de microgranules comportant une âme neutre, au moins une première couche intermédiaire comprenant au moins un alcaloïde appartenant au groupe des β-carbolines quaternaires du type serpentine, alstonine, sempervirine et flavopéreirine puis au moins une couche extérieure constituée par une enveloppe microporeuse comprenant au moins un polymère nature et/ou synthétique du type gomme laque, gomme arabique, gélatine, cellulose, éthyl cellulose, acétophtalate de cellulose, triacétate de cellulose, polyvinylpyrrolidone, polyoxyéthylèneglycol, méthacrylates et copolymères styrène-acrylonitrile.

2. Compositions pharmaceutiques selon la revendication 1, caractérisées en ce que les β-carbolines quaternaires sont utilisées sous forme galénique à libération prolongée à raison de 2 mg à 500 mg par prise.

3. Compositions selon une des revendications 1 ou 2, utiles comme agents anti-tumoraux, caractérisées en ce qu'elles sont associées avec une substance anti-tumorale classique de cyclophosphamide selon une proportion comprise entre 1 et 2 parties en poids pour 10 parties en poids de β-carboline quaternaire.


**Claims**

1. Pharmaceutical compositions having preferential action on the replication of DNA and the synthesis RNA in tumorous, cancerous and viral cells, characterized in that they consist of microgranules having a neutral central core, at least one first intermediary layer comprising at least one alkaloid of the group of beta-carbolines of the type serpentine, alstonine, sempervirine and flavopereirine, and at least an outer layer made up by a microporous envelope, comprising at least a natural and/or synthetic polymer of the type : shellac, arabic gum, gelatine, cellulose, ethyl cellulose, cellulose acetophtalate, cellulose triacetate, polyvinyl pyrrolidone, polyoxyethylene glycol, methacrylates and styrene-acrylonitrile copolymers.

2. Pharmaceutical compositions according to claim 1, characterized in that the quarternary beta-carbolines are used in the galenic form having prolonged release in an amount from 2 mg to 500 mg per individual dose.

3. Compositions according to one of the claims 1 or 2, useful as anti-tumor agents, characterized in that they are associated with one conventional anti-tumoral substance of the cyclophosphamide type in a proportion lying between 1 and 2 parts by weight to 10 parts by weight of quaternary beta-carboline.


**Patentansprüche**

1. Pharmazeutische Zusammensetzungen mit bevorzugter Wirkung auf die Replication der DNS und die Synthese der RNS von Tumor-, Krebs- und Viruszellen, dadurch gekennzeichnet, daß sie aus Mikrokügelchen bestehen, die einen neutralen Kern, mindestens eine erste Zwischenschicht, die mindestens ein zur Gruppe der quatären β-Carboline gehörendes Alkaloid vom Serpentin-, Alstonin-, Sempervirin- und Flavopereirintyp enthält, dann mindestens eine Außenschicht umfaßt, die durch eine mikroporöse Umhüllung gebildet ist, die mindestens ein Natur- und/oder synthetisches Polymer vom Typ Gummilack, gummi arabicum, Gelatine, Zellulose, Äthylzellulose, Zelluloseacetophtalat, Zellulosetriacetat, Polyvinylpyrrolidon, Polyoxyäthylenglykol, Methacrylsäureester und Styrol-Acrylnitril-Copolymere enthält.

2. Pharmazeutische Zusammensetzungen nach Anspruch 1, dadurch gekennzeichnet, daß die quatären β-Carboline in galenischer Form zur verlängerten Freisetzung im Verhältnis von 2 mg zu 500 mg je Einnahme verwendet werden.

3. Zusammensetzungen nach einem der Ansprüche 1 oder 2 verwendbar als Antitumormittel, dadurch gekennzeichnet, daß sie mit einer klassischen Cyclophosphamid-Antitumorsubstanz in einem Verhältnis eingeschlossen zwischen 1 und 2 Gewichtsteilen auf 10 Gewichtsteile von quatärem β-Carbolin verbunden sind.

Fig:1

Fig:2